# EUROPÄISCHE PATENTSCHRIFT

(11) **EP 1 014 939 B1**
(45) Veröffentlichungstag und Bekanntmachung des Hinweises auf die Patenterteilung: **03.11.2004**
(21) Anmeldenummer: 98936295.9
(22) Anmeldetag: 28.05.1998
(51) Int. Cl.: A61K 9/00

(54) **EINKAPSELN VON WASSERUNLÖSLICHEN WIRKSTOFFEN MIT AMPHIPHILEM CHARAKTER**
ENCAPSULATION OF WATER INSOLUBLE ACTIVE SUBSTANCES WITH AN AMPHIPHILIC CHARACTER
ENCAPSULAGE DE PRINCIPES ACTIFS NON HYDROSOLUBLES PRESENTANT UN CARACTERE AMPHIPHILE

(30) Priorität: 28.05.1997 DE 19722405
(43) Veröffentlichungstag der Anmeldung: 05.07.2000
(73) Patentinhaber: IFAC Institut für angewandte Colloidtechnologie GmbH & Co. KG, 47138 Duisburg (DE)
(72) Erfinder: DAHMS, Gerd, H., D-47138 Duisburg (DE)
(74) Vertreter: Isenbruck, Günter, Dr.
(86) Internationale Anmeldenummer: PCT/EP1998/003201
(87) Internationale Veröffentlichungsnummer: WO 1998/053797

(56) Entgegenhaltungen:
- DE-A- 3 224 619
- FR-A- 2 735 658
- CHEMICAL ABSTRACTS, vol. 98, no. 20, 16. Mai 1983 Columbus, Ohio, US; abstract no. 166907, TAIHO PHARMACEUTICAL CO., LTD., JAPAN ET AL: "Pharmaceuticals containing fatty acid polyglycerol esters" XP002082143 & JP 58 013508 A (TAIHO PHARMACEUTICAL CO., LTD., JAPAN;FUROINTO SANGYO K. K.)

## Beschreibung

Die Erfindung betrifft eingekapselte wasserunlösliche Wirkstoffe mit amphiphilem Charakter, insbesondere wasserunlösliche Vitamine, mit erhöhter Stabilität in wäßrigen Systemen, ein Verfahren zu ihrer Herstellung und ihre Verwendung.

In der Praxis der Medizin, Landwirtschaft, aber auch Kosmetik - heute häufig gemeinsam als "Life Sciences" bezeichnet - besteht eine große Nachfrage nach Wirkstoff-Formen (Formulierungen), die in wäßrigen oder wasserhaltigen Systemen stabil gelagert oder in solche stabil eingebracht werden können. Typische Hilfsmittel für solche Systeme, die z.B. als Wasser-in-Öl- oder Öl-in-Wasser-Emulsionen vorliegen, sind Tenside (oberflächenaktive Verbindungen), die auf diesem Gebiet Dispergierhilfsmittel genannt werden. Als Dispergierhilfsmittel geeignete Verbindungen weisen im allgemeinen im Molekül einen hydrophilen und einen hydrophoben Bestandteil auf; Beispiele sind Reaktionsprodukte (Ester, Ether) aus langkettigen Carbonsäuren (z.B. C₈- bis C₂₀-Säuren) oder langkettigen Alkoholen einerseits mit Hydroxylgruppen aufweisenden oder bildenden Verbindungen wie Glycerin, Ethylenoxid, Propylenoxid, Milchsäure oder Weinsäure andererseits, die auch in oligomeren oder polymeren Formen eingesetzt werden können oder unter den gewählten Bedingungen entstehen.

Es hat sich dabei herausgestellt, daß bei den heutigen ökologischen, ökonomischen, insbesondere aber auch physiologischen (z.B. dermatologischen) Anforderungen es kaum Systeme und Kompositionen gibt, die generell für jeden Wirkstoff und jedes Anwendungsgebiet geeignet sind. In jüngster Zeit hat sich auch ein Arbeitsgebiet entwickelt, das versucht, die oberflächenaktiven Eigenschaften der genannten Tenside gezielt dazu zu verwenden, wasserunlösliche Wirkstoffe einzukapseln und damit zusätzlich auch zu schützen.

In der US 2,650,895 werden Vitaminemulsionen beschrieben, in denen lipophile Vitamine (z.B. Vitamin A, B oder D) vorliegen, die durch Einbringen in ein wäßriges System erzeugt werden, das eine eßbare Substanz (wie Dextrine oder Gelatine) enthält, und wonach diese Emulsion sprühgetrocknet wird, so daß feine Teilchen entstehen, die von der eßbaren Substanz eingekapselt sind. Allerdings benötigen dieses Vitamine einen lipophilen Träger, z.B. aus Öl, Lecithin, Alkohol und Diethanolamin.

Eine ähnlich komplexe Vorgehensweise mit Sprühtrocknung wird auch in der JP-A 85/042317 (Derwent-Zitat 85-095332) beschrieben, wobei die umhüllende Substanz ein Protein (z.B. Soja) oder ein teilhydrolysiertes Protein ist und als umhüllte Substanz auch Vitamine genannt werden.

In der Veröffentlichung von J.W.Cook "Acyl Lactylates - Applications for a Multi-Functional Group of α-Hydroxy Acid Derivatives" in The Cosmetics and Toiletries Manufacture Worldwide 1995 Annual (Acyl-Lactylate - Anwendungen für eine multifunktionelle Gruppe von α-Hydroxy-Carbonsäurederivaten) werden verschiedenste Anwendungen/Rezepturen für die Salze der Ester aus langkettigen Carbonsäuren (speziell C₈- bis C₁₈-Säuren) und 1 bis 3 Molen Milchsäure aufgeführt. Es werden Duschgele, Lotionen, Seifenstücke, Sonnenmilch, Badeöle und Haarfestiger genannt. Eine gezielte Anwendung zur Verkapselung wasserunlöslicher Wirkstoffe wird aber nicht erwähnt.

Weitere aus dem Stand der Technik bekannte Tensidester leiten sich (FR-A-2 735 658) von längerkettigen Carbonsäuren wie Palmitinsäure (C₁₆), Stearinsäure (C₁₈), Oleinsäure (C₁₈), Linolsäure (C₁₈) oder Arachidonsäure (C₂₀) ab oder (DE-A 32 24 619 oder JP-A 58/013 508) von Ölsäure/Oleinsäure (C₁₈). EP-A-0 582 503 befasst sich mit Verkapselung von Wirkstoffen mit amphiphilem Charakter.

Aufgabe der vorliegenden Erfindung ist es, ein System an eingekapselten, an sich wasserunlöslichen Wirkstoffen mit amphiphilem Charakter zu entwickeln, das die Wirkstoffe nicht nur zusätzlich gegenüber Umwelteinflüssen (z.B. Oxidation, Verfärbung o.ä.) schützt, sondern auch ermöglicht, diese so eingekapselten Wirkstoffe stabil in wäßrige Zusammensetzungen einzubauen bzw. in diesen stabil zu lagern.

Die Lösung dieser Aufgabe sind eingekapselte, wasserunlösliche Wirkstoffe mit amphiphilem Charakter enthaltend mindestens ein Vitamin aus der Gruppe Vitamin C-Ester, Retinol und Tocopherol oder Ceramide, mit einem Gehalt an Wasser und mindestens einem Tensid aus der Gruppe der Ester von langkettigen Carbonsäuren mit Hydroxylgruppen enthaltenden Carbonsäuren oder deren Salzen und der Ester von langkettigen Carbonsäuren mit Polyalkoholen. Dabei sind die langkettigen Carbonsäuren C₈ bis C₁₂-Säuren.

Von den in den Wirkstoffen mit amphiphilem Charakter (nachfolgend nur noch "Wirkstoffe") enthaltenen Vitaminen bzw. ausschließlich als Wirkstoff vorliegenden Vitaminen aus der Gruppe Vitamin C - Ester, insbesondere Vitamin C-Palmitat, Retinol und Tocopherol ist bekannt, daß sie hydrophoben amphiphilen Charakter zeigen. Im übrigen ist unter dem Begriff wasserunlöslicher Wirkstoff bzw. wasserunlösliches Vitamin zu verstehen, daß nicht nur der Stoff selbst bereits wasserunlöslich sein muß, sondern daß er auch an sich wasserlöslich sein kann (wie Vitamin C = Ascorbinsäure), aber durch spezielle Behandlung wasserunlöslich gemacht worden ist (wie Veresterung zu Vitamin C-Palmitat). Unter amphiphil soll dabei verstanden werden, daß die Wirkstoffmoleküle sowohl hydrophile als auch lipophile Eigenschaften aufweisen.

Zur chemischen Charakterisierung der bevorzugt eingesetzten Vitamine wird beispielhaft auf Römpp Chemie Lexikon, 9. Auflage, 1989-1992, Georg Thieme Verlag Stuttgart, Stichwörter Retinol, Tocopherole und Vitamine verwiesen.

Die anderen erfindungsgemäß einsetzbaren Wirkstoffe sind Ceramide, d.h. lipophile Amide aus einem gesättigten und ungesättigten Aminodiolrest (Dihydrosphingosine und Sphingosine) und gesättigten Aminotriolrest (Phytosphingosine), langkettigen Fettsäureresten und langkettigen Alkylresten im Aminodiolteil (siehe z.B. Stichwort "Ceramide" und "Cerebroside" in Römpp).

Den erfindungsgemäß einsetzbaren Wirkstoffen ist gemeinsam, daß sie in nicht eingekapselter Form entweder in Formulierungen nicht stabil oder zumindest nicht über längere Zeiträume lagerfähig sind.

Bei der Untersuchung der erfindungsgemäB eingekapselten Wirkstoffe scheint sich herauszustellen, daß angenommen werden kann, daß die Wirkstoffe in Membranen von liposomalen Bläschenstrukturen der Tenside eingebaut (eingekapselt) werden. Als liposomale Vesikel beeinhalten die Kapseln bzw. eingekapselten Wirkstoffe Wasser. Erfindungsgemäß handelt es sich um eine "weiche" Verkapselung der Wirkstoffe. Die Kapsel besteht hierbei aus, vorzugsweise hochviskosen, Membranschichten, die sowohl die Tenside als auch die Wirkstoffe beinhalten.

Zwischen den einzelnen Membranschichten liegt Wasser in gebundener Form vor. Im Gegensatz zu den ähnlich aufgebauten Liposomen enthalten diese Membrankapseln vorzugsweise keine Phospholipide. Während davon ausgegangen wird, daß Lecithin als Grundkörper für Liposome verwendet werden muß, zeigt die vorliegende Erfindung, daß bei den darin verwendeten Tensiden ganz auf Lecithin verzichtet werden kann. Die Form der Kapseln ist nach Untersuchungen mit dem Elektronenmikroskop auch nicht wie bei Liposomen sphärisch, sondern stark elipsoid ausgebildet.

Die Menge an eingekapseltem Wirkstoff hängt dabei sowohl von der Struktur des Wirkstoffes selbst, aber insbesondere von der Struktur des Tensides ab. Die Ergebnisse werden mit Tensiden erzielt, deren langkettige Carbonsäurebausteine sich von C₈- bis C₁₂-Säuren (d.h. von Capryl- bis Laurinsäure), insbesondere von C₁₀-Säure (d.h. von Caprinsäure), ableiten; in diesen Tensiden ist der Hydroxylgruppen enthaltende Baustein bevorzugt ein Monomeres, Dimeres oder Trimeres der Milchsäure oder eines ihrer Salze oder einer vergleichbaren Hydroxycarbonsäure, insbesondere ein Dimeres der Milchsäure in Salzform (d.h. ein Lactylat), oder ein Oligomeres aus 2 bis 10 Molekülen Glycerin (Polyglycerin), wobei im Tensid auf ein Mol Polyglycerin insbesondere 2 bis 3 Mol Carbonsäure kommen.

Die Grenze zur Herstellung praxisgerechter, d.h. stabiler, eingekapselter wasserunlöslicher Wirkstoffe liegt in möglichen Konkurrenz- oder Ausweichreaktionen der Verbindungskombination aus Wirkstoff und Tensid; häufig führen diese Reaktionen zu stabilen flüssigkristallinen (lamellaren) Strukturen oder zu netzwerkartigen Gelstrukturen. Um eine gute Umkapselung des Wirkstoffes zu erreichen, müssen beide Molekülarten - Wirkstoff und Tensid - gemeinsam so angeordnet bleiben, daß sie z.B. Bläschen bilden können; diese gute Umkapselung ist sehr wichtig, um den Wirkstoff in der Praxisanwendung stabil (z.B. unversehrt) erhalten zu können.

Wenn die erfindungsgemäß eingekapselten Wirkstoffe, z.B. auf dem Anwendungsgebiet Kosmetik, in Detergentiensysteme eingearbeitet werden sollen, beispielsweise in wäßrige, Betain- oder Na-laurylethersulfat enthaltende Systeme, so kann ihre Stabilität in diesen Systemen durch Zusatz von wasserlöslichen Polymeren wie Carboxyvinylpolymeren oder von Cyclodextrinderivaten noch verbessert werden. Eine ähnliche, stabilisierende Wirkung, die möglicherweise durch Reduzierung der Teilchengröße der eingekapselten Wirkstoffe erreicht wird, kann auch mit Lecithin-Zusätzen bewirkt werden.

Darüber hinaus ist es durch Variation in der Kettenlänge der Fettsäureanteile in der Tensidkomponente der eingekapselten Wirkstoffe möglich, den Schmelzpunkt der eingekapselten Wirkstoffe in wäßrigen Systemen zu variieren, z.B. zwischen 25 und 50°C. Dadurch kann bei der späteren Applikation, z.B. auf der Haut, eine verbesserte Penetration erreicht werden.

Im übrigen ist die Herstellung der eingekapselten Wirkstoffe einfacher als im Stand der Technik (z.B. solchen auf Lecithinbasis), da kein hoher Rührenergieaufwand mehr erforderlich ist, so daß oftmals auf aufwendige Homogenisatoren oder Hilfstoffzusätze (z.B. Alkohole) verzichtet werden kann. Die Bildung der eingekapselten Wirkstoffe erfolgt nahezu in situ, d.h. auch bei minimalem Rühraufwand, etwa durch Rühren von Hand mit einem Spatel, werden Partikel mit einer Größe im Nanometerbereich gebildet. Dabei können derart feine Kapseln erzeugt werden, daß das erhaltene Gemisch nicht mehr wie bei der herkömmlichen Liposomherstellung emulsionsartig, sondern vollständig klar erscheint. Auch auf die Konservierung der eingekapselten Wirkstoffe kann häufig verzichtet werden, da beispielsweise Lactylate antimikrobielle Eigenschaften zeigen.

Gemäß der vorliegenden Erfindung ist die Beladung der Membran mit amphiphilen Wirkstoffen weitaus effizienter und flexibler verglichen mit herkömmlichen membranartigen Systemen wie z.B. den Liposomen. So können insbesondere hoch schmelzende Wirkstoffe wie Ceramide, Vitamin C-Palmitat oder Vitamin D₃, die als äußerst schwierig zu verkapsulieren gelten, auf einfach Weise in hohen Anteilen von vorzugsweise mehr als 30 % in die Membran eingeschlossen werden.

Eine weitere Lösung der gestellten Aufgabe ist ein Verfahren zur Herstellung der erfindungsgemäßen eingekapselten, wasserunlöslichen Wirkstoffe, in dem a) die Komponenten Wirkstoff und Tensid zu einer homogenen Flüssigkeit vermischt und b) dieses Gemisch mit Wasser vesetzt und erneut homogen vermischt und auf Raumtemperatur abgekühlt wird. Die vorstehend erwähnten stabilisierenden Zusätze können dem Wasser zugegeben werden. Die erste Stufe a) wird im allgemeinen bei einer Temperatur von >50°C durchgeführt, die zweite bei <50°C. Besonders bevorzugt wird die erste Stufe a) bei einer Temperatur im Bereich von 55 bis 80°C durchgeführt. Auch die zweite Stufe b) kann bei dieser Temperatur durchgeführt werden. Die bevorzugten Mengenrelationen liegen bei 0,1 bis 15, besonders bevorzugt 0,3 bis 12, insbesondere 0,5 bis 10 Gew.-Teilen Wirkstoff auf 1 bis 5 Gew.-Teile Tensid und bei 0,1 bis 15 Gew.-Teilen Wirkstoff/Tensid-Gemisch auf 1 bis 5 Gew.-Teile Wasser.

Die nachfolgenden Beispiele erläutern die Erfindung zusätzlich.

### Beispiele

Um eine homogene Verteilung des Wirkstoffs zu erreichen, wird dieser gemeinsam mit dem Tensid auf 80°C unter Rühren erhitzt und bei 75 bis 80°C so lange vermischt, bis eine vollständig klare Lösung erzielt wird.

Nach dem Abkühlen auf 25°C wird das Gemisch auf etwa 40°C erhitzt und Wasser hinzugefügt und intensiv gerührt, bis das System vollständig homogenisiert ist. Anschließend wird das System mikroskopisch untersucht, um das Nicht-Vorhandensein von kristallinen Phasen auszuschließen. Die Versuche werden in 50 ml Gefäßen durchgeführt.

### Beispiel 1

Es wird ein Gemisch aus 90 Gew.-% Vitamin C-Monopalmitat und 10 Gew.-% Polyglycerin-10-trilaurat (®Decaglyn 3L der Nikko Chemicals Co., Ltd, Japan) erzeugt.

### Beispiel 2

Es wird ein Gemisch aus 70 Gew.-% Vitamin C-Monopalmitat und 30 Gew.-% Polyglycerin-10-trilaurat erzeugt.

### Beispiel 3

30 Gew.-% des Gemisches aus Beispiel 1 oder 2 werden mit 70 Gew.-% demineralisiertem Wasser versetzt. Der erhaltene verkapselte Wirkstoff besteht mikroskopisch aus stabilen, stark elipsoiden liposomalen Bläschen, die über 2 Monate lagerstabil sind.

### Beispiel 4

Es wird nach Beispiel 1 verfahren, aber mit Polyglycerin-10-tricaprat (®Decaglyn 3D).

### Beispiel 5

Es wird nach Beispiel 2 verfahren, aber ebenfalls mit ®Decaglyn 3D.

### Beispiel 6

Es wird nach Beispiel 3 verfahren, aber mit den Gemischen aus Beispielen 4 und 5. Der erhaltene verkapselte Wirkstoff besteht ebenfalls aus stabilen Bläschen, die über 2 Monate lagerstabil sind.

### Beispiel 7

Um auch Kombinationen verschiedener Wirkstoffe zu untersuchen, werden 10 Gew.-% Vitamin C-Dipalmitat und 80 Gew.-% ®Decaglyn 3D unter Rühren auf 80°C erhitzt, gerührt, bis eine klare Lösung entsteht, und dann auf 50°C abgekühlt. In dieses erste Gemisch werden 10 Gew.-% Retinol homogen eingemischt. Das zweite Gemisch wird auf 25°C abgekühlt, und davon werden 20 Gew.-Teile mit 80 Gew.-Teilen demineralisiertem Wasser versetzt. Das Ergebnis sind stabile Bläschen.

### Beispiel 8

Es wird nach Beispiel 7 verfahren, aber unter Verwendung von Tocopherol im zweiten Gemisch. Das Ergebnis sind stabile Bläschen.

### Beispiel 9

Es wird nach Beispiel 7 verfahren, aber unter Verwendung von 5 Gew.-% Vitamin C Dipalmitat, 85 Gew.-% ®Decaglyn 3D und je 5 Gew.-% Retinol und Tocopherol. Das Ergebnis sind stabile Bläschen.

### Beispiel 10

Es wird nach Beispiel 1 und Beispiel 3 verfahren, aber unter Verwendung von dem Tensid ®Pationic 138C der R.I.T.A. Corporation, Woodstock II/USA, einem Natriumsalz des Lauroyllactylats. Das Ergebnis sind stabile Bläschen.

### Beispiel 11

Ein Gemisch aus 30 Gew.-% Ceramid IIIB (von Cosmoferm, NL) wird mit 30 Gew.-% Natriumcaproyllactylat und 40 Gew.-% Polyglycerin-Dicaprat bei 90°C gemischt, bis eine homogen-klare Lösung erhalten wird. Zu dieser Lösung wird demineralisiertes Wasser unter Rühren mit einem Spatel zugegeben. Dabei bilden sich spontan bei einem Wassergehalt von etwa 80 Gew.-% feinste Vesikel von einer Größe unterhalb 100 nm.

### Beispiel 12

Ein Gemisch aus 25 Gew.-% Ceramid III (von Cosmoferm, NL) wird mit 75 Gew.-% Natriumlauroyllactylat bei 90°C gemischt, bis eine homogen klare Lösung erhalten wird. Zu dieser Lösung wird demineralisiertes Wasser unter Rühren mit einem Spatel zugegeben. Dabei bilden sich spontan bei einem Wassergehalt von etwa 80 Gew.-% feinste Vesikel von einer Größe unterhalb 100 nm.

Mit herkömmlicher Liposomtechnologie konnten mit den in den Beispielen 11 und 12 angegebenen Mengen keine Verkapsulierungen mit Ceramiden hergestellt werden.

## Patentansprüche

1. Eingekapselte, wasserunlösliche Wirkstoffe mit amphiphilem Charakter, enthaltend mindestens ein Vitamin aus der Gruppe Vitamin C-Ester, Retinol und Tocopherol oder Ceramide, mit einem Gehalt an Wasser und mindestens einem Tensid aus der Gruppe der Ester von langkettigen Carbonsäuren mit Hydroxylgruppen enthaltenden Carbonsäuren oder deren Salzen, die sich von Milchsäure oder deren Di- oder Trimeren ableiten und der Ester von langkettigen Carbonsäuren mit Polyalkoholen, die sich von den Oligomeren des Glycerin ableiten, wobei die langkettigen Carbonsäuren C₈ bis C₁₂-Säuren sind.

2. Eingekapselte Wirkstoffe nach Anspruch 1, **dadurch gekennzeichnet**, däß sie zusätzlich einen Gehalt an wasserlöslichen Polymeren, Cyclodextrinderivaten oder Lecithin aufweisen.

3. Eingekapselte Wirkstoffe nach Anspruch 1 oder 2, **dadurch gekennzeichnet, daß** die Kapseln aus Membranschichten aufgebaut sind, die sowohl das mindestens eine Tensid als auch den mindestens einen Wirkstoff enthalten, und elipsoid sein können.

4. Verfahren zur Herstellung eingekapselter, wasserunlöslicher Wirkstoffe nach einem der Ansprüche 1 bis 3 **dadurch gekennzeichnet**, däß a) die Komponenten Wirkstoff und Tensid zu einer homogenen Flüssigkeit vermischt und b) dieses Gemisch mit Wasser, gegebenenfalls mit einem Gehalt an wasserlöslichen Polymeren, Cyclodextrinderivaten oder Lecithin, versetzt und erneut homogen vermischt und auf Raumtemperatur abgekühlt wird.

5. Verfahren nach Anspruch 4, **dadurch gekennzeichnet, daß** das Vermischen in Schritt a) bei einer Temperatur im Bereich von 55 und 80°C erfolgt.

6. Verwendung der eingekapselten Wirkstoffe nach einem der Ansprüche 1 bis 3 bei der Zubereitung von pharmazeutischen, agrochemischen oder kosmetischen Formulierungen.

## Claims

1. Encapsulated, water-insoluble active compounds having amphiphilic character, comprising at least one vitamin from the group consisting of vitamin C esters, retinol and tocopherol or ceramides, containing water and at least one surfactant from the group consisting of the esters of long-chain carboxylic acids with carboxylic acids comprising hydroxyl groups or their salts which are derived from lactic acid or its di- or trimers and of the esters of long-chain carboxylic acids with polyalcohols which are derived from the oligomers of glycerol, the long-chain carboxylic acids being C₈ to C₁₂ acids.

2. Encapsulated active compounds according to Claim 1, **characterized in that** they additionally contain water-soluble polymers, cyclodextrin derivatives or lecithin.

3. Encapsulated active compounds according to Claim 1 or 2, **characterized in that** the capsules are constructed of membrane layers which contain both the at least one surfactant and the at least one active compound, and can be ellipsoidal.

4. Process for the preparation of encapsulated, water-insoluble active compounds according to one of Claims 1 to 3, **characterized in that** a) the components active compound and surfactant are mixed to give a homogeneous liquid and b) this mixture is treated with water, optionally containing water-soluble polymers, cyclodextrin derivatives or lecithin, and is again homogeneously mixed and cooled to room temperature.

5. Process according to Claim 4, **characterized in that** the mixing in step a) is carried out at a temperature in the range from 55 to 80°C.

6. Use of the encapsulated active compounds according to one of Claims 1 to 3 in the preparation of pharmaceutical, agrochemical or cosmetic formulations.

## Revendications

1. Substances actives insolubles dans l'eau avec un caractère amphiphile, encapsulées, contenant au moins une vitamine du groupe consistant en ester de vitamine C, rétinol et tocophérol ou céramide, avec une quantité d'eau et au moins un agent tensioactif du groupe des esters d'acides organiques à longues chaînes avec des acides organiques contenant des groupements hydroxyles ou leurs sels, qui dérivent de l'acide lactique ou de leurs di- ou tri-mères et du groupe des esters d'acides organiques à longues chaînes avec des polyalcools, qui dérivent des oligomères de la glycérine, dans lesquels les acides organiques à longues chaînes sont des acides en C₈ à C₁₂.

2. Substances actives encapsulées selon la revendication 1, **caractérisées en ce qu'**elles présentent éventuellement une quantité de polymères solubles dans l'eau, de dérivés de cyclodextrine ou de lécithine.

3. Substances actives encapsulées selon la revendication 1 ou 2, **caractérisées en ce que** les capsules sont constituées de couches de membrane, qui contiennent le au moins un tensioactif et la au moins une substance active, et qui peuvent être ellipsoïdes.

4. Procédé de fabrication de substances actives encapsulées, insolubles dans l'eau, selon l'une des revendications 1 à 3, **caractérisé en ce que** a) les constituants substance active et tensioactif sont mélangés pour obtenir un fluide homogène et b) ce mélange est de nouveau mélangé de façon homogène avec de l'eau, remplacée le cas échéant par une quantité de polymères solubles dans l'eau, de dérivés de cyclodextrine ou de lécithine et refroidi à température ambiante.

5. Procédé de fabrication selon la revendication 4, **caractérisé en ce que** le mélange de l'étape a) a lieu à une température située dans la plage allant de 55 à 80°C.

6. Utilisation des substances actives encapsulées selon l'une des revendications 1 à 3 dans la préparation de formulations pharmaceutiques, agrochimiques ou cosmétiques.
